# EUROPÄISCHE PATENTSCHRIFT

(11) **EP 2 228 432 B1**
(45) Veröffentlichungstag und Bekanntmachung des Hinweises auf die Patenterteilung: **23.11.2011**
(21) Anmeldenummer: 09154581.4
(22) Anmeldetag: 08.03.2009
(51) Int. Cl.: C12M 1/00, B01F 3/04, C12M 1/08, B01J 19/24

(54) **Bioreaktor und Verfahren zum Betrieb eines Bioreaktors**
Bioreactor and method for operating same
Bioréacteur et procédé destiné au fonctionnement d'un bioréacteur

(43) Veröffentlichungstag der Anmeldung: 15.09.2010
(73) Patentinhaber: SSC Strategic Science Consult GmbH, 22761 Hamburg (DE)
(72) Erfinder: Kerner, Martin, 22761 Hamburg (DE)
(74) Vertreter: UEXKÜLL & STOLBERG

(56) Entgegenhaltungen:
- WO-A-91/07080
- DE-A1- 19 916 597
- GB-A- 1 405 264
- JP-A- 54 055 780

## Beschreibung

Die Erfindung betrifft einen Bioreaktor zur Kultivierung phototropher Organismen und ein Verfahren zum Betreiben eines derartigen Bioreaktors zur Kultivierung phototropher Organismen.

Phototrophe Organismen, zu denen ein- und wenigzellige Mikroorganismen wie beispielsweise Algen, Cyanobakterien und pflanzliche Zellkulturen gehören, zeichnen sich dadurch aus, dass sie mittels Photosynthese Licht als Energiequelle nutzen und damit Biomasse aufbauen. Ihre Kultivierung erfolgt mit dem Ziel, sie selbst (Biomasse) oder Stoffwechselprodukte von ihnen zu produzieren. Dazu werden auch als Photobioreaktoren bezeichnete Bioreaktoren eingesetzt. Solche Bioreaktoren weisen allgemein ein lichtdurchlässiges Gehäuse auf, das einen Reaktorraum umschließt. In dem Reaktorraum werden die jeweiligen Organismen in einem Kultur- bzw. Nährmedium angeordnet, und anschließend wird Licht durch das Reaktorgehäuse in den Reaktorraum eingestrahlt.

Dabei besteht das grundsätzliche Problem, dass die Lichtintensität aufgrund der Lichtabsorption durch die Organismen und in dem Kulturmedium und durch Abschattungseffekte mit zunehmender Eindringtiefe in das Kulturmedium stark abnimmt, so dass eine ausreichende Lichtzufuhr lediglich in einer gewissen Schicht in der Nähe der belichteten Gehäusebereiche gewährleistet ist. Darüber hinaus ist auch zu beachten, dass phototrophe Organismen einfallendes Licht nicht kontinuierlich verarbeiten können, sondern theoretisch sogar nur während eines Drittels der Zeit Licht aufnehmen und für die Energiegewinnung nutzen können. Aus diesem Grund wird eine optimale Lichtausnutzung und Photosyntheseaktivität der phototrophen Organismen erreicht, wenn die Organismen im statistischen Mittel einen Hell-Dunkel-Zyklus durchlaufen, so dass sie während eines ersten Zeitraums Licht relativ hoher Intensität aufnehmen und anschließend für einen zweiten Zeitraum in relative Dunkelheit zurückkehren.

Auf diese Weise können zum einen mehr Organismen an der phototrophen Aktivität teilnehmen, und zum anderen sind die Organismen nur für kurze Zeit maximaler Lichtintensität ausgesetzt, die bei Starklicht sogar zu einer Schädigung der Organismen (sog. Photoinhibition) führen kann.

Im Stand der Technik sind verschiedene Mechanismen bekannt, mit denen versucht worden ist, den genannten Aspekten möglichst optimal Rechnung zu tragen. Ein in dem Dokument DE 199 16 597 A1 beschriebener Bioreaktor basiert auf dem Prinzip der sog. Airlift- oder Airlift-Schlaufen-Bioreaktoren. Bei diesem Bioreaktortyp wird das Kulturmedium in einer vorgegebenen Schlaufe in Bewegung versetzt, indem auf einer Seite Gas in den Bioreaktor eingedüst wird, das dann in dem Kulturmedium aufsteigt.

Der aus DE 199 16 597 A1 bekannte Bioreaktor weist zwei symmetrisch innerhalb eines quaderförmigen Reaktorraums angeordnete Leitplatten oder Zwischenwände auf, die parallel zueinander und zu zwei gegenüberliegenden Seitenwänden des Bioreaktorgehäuses verlaufen und sich über einen Großteil der Länge des Reaktorraums erstrecken. Die Zwischenwände, die jeweils an ihren beiden Enden von den benachbarten Stirnwänden des Reaktorgehäuses beabstandet sind, bilden zwischen sich einen zentralen Strömungsleitkanal, und zwischen jeder Zwischenwand und der benachbarten Seitenwand des Reaktorgehäuses wird je ein äußerer Strömungsleitkanal gebildet. Das Gas wird an einer Seite des Bioreaktors durch die entsprechende Stirnwand in der Weise in den Reaktorraum eingedüst, dass das Gas durch den zentralen Strömungsleitkanal in dem Kulturmedium nach oben steigt, mit dem der Reaktorraum gefüllt ist. Auf diese Weise entsteht eine Flüssigkeitsströmung, die in dem zentralen Strömungsleitkanal dem Gasstrom folgt und in den äußeren Strömungsleitkanälen entgegengerichtet ist, d.h. es existieren zwei in Form einer Acht verbundene Schlaufen.

Die Einstrahlung von Licht in den Reaktorraum erfolgt von einer Seite parallel zu den Zwischenwänden und senkrecht zur Erstreckungsrichtung der Strömungsleitkanäle und damit auch senkrecht zu der Flüssigkeitsströmung in den Strömungsleitkanälen. Um eine Bewegung der Organismen zu erreichen, durch die sie im statistischen Mittel einen Hell-Dunkel-Zyklus durchlaufen, wird die beschriebene Flüssigkeitsströmung turbulent geführt. Zur Erhöhung der Turbulenz wird zum einen der Einbau statischer Mischer bzw. von Strombrechern und zum anderen eine Erhöhung der Begasungsrate vorgeschlagen. Auf diese Weise sollen im gesamten Reaktorraum dreidimensionale Turbulenzen erzeugt werden, wobei angestrebt wird, für die Organismen einen Hell-Dunkel-Zyklus mit einer Frequenz von mehr als 1 Hz bereitzustellen. In dem Reaktorraumabschnitt zwischen der Stirnwand des Reaktorgehäuses, die der Stirnwand gegenüberliegt, durch die die Begasung erfolgt, und den benachbarten Enden der Zwischenwände befindet sich ein kleiner hochturbulenter Kopfraum, der für den Gasaustausch und die Temperierung ausgenutzt wird.

Es hat sich jedoch gezeigt, dass die durch den bekannten Bioreaktor erzielbare Turbulenz nicht groß genug ist, um optimale Frequenzen für den Hell-Dunkel-Zyklus und damit eine optimale Lichtausnutzung zu erzielen und um einen Bewuchs der Reaktoroberflächen mit Algen zu verhindern (sog. Biofouling), was wiederum den Lichteintritt in den Reaktorraum hemmt. Ferner sind der für eine verbesserte Lichtausnutzung erstrebenswerten Vergrößerung des Verhältnisses von beleuchtbarer Gehäuseseitenfläche zu Reaktorraumvolumen bei den bekannten Bioreaktoren aufgrund ihrer zur Ausbildung einer hohen Turbulenz erforderlichen Mindestdicke Grenzen gesetzt, an denen noch Verbesserungsbedarf besteht. Außerdem muss der Bioreaktor in senkrechter Position betrieben werden, d.h. in einem Winkel von 90° zur Aufstellungsfläche, so dass eine Ausrichtung der beleuchteten Reaktorwandung in Bezug auf die Sonne zur Maximierung des Lichteintritts und Minimierung der Reflexion nicht möglich ist.

Es ist Aufgabe der vorliegenden Erfindung, einen Bioreaktor zur Kultivierung phototropher Organismen und ein Verfahren zum Betrieb eines solchen Bioreaktors bereitzustellen, durch die die Lichtausnutzung durch die Organismen weiter verbessert wird.

Diese Aufgabe wird durch einen Bioreaktor mit den Merkmalen des Anspruchs 1 und durch ein Verfahren mit den Merkmalen des Anspruchs 11 gelöst. Bevorzugte Ausführungsformen des Bioreaktors und des Verfahrens sind Gegenstand der jeweils zugehörigen Unteransprüche.

Nach der vorliegenden Erfindung ist vorgesehen, dass ein Bioreaktor zur Kultivierung phototropher, in einem Reaktorraum des Bioreaktors angeordneter Organismen ein lichtdurchlässiges quader- und plattenförmiges Gehäuse aufweist, in dessen Innerem der Reaktorraum angeordnet ist. Das Gehäuse ist demnach flach und ist auf zwei gegenüberliegenden Seiten von je einer Seitenwand mit einer im Verhältnis zur Dicke sehr viel ausgedehnteren bzw. größeren Fläche begrenzt. Neben diesen beiden Seitenwänden sind noch zwei weitere einander gegenüberliegende Seitenwände mit einer kleineren Fläche und zwei einander gegenüberliegende Stirnwände vorhanden. Aufgrund der plattenförmigen Ausgestaltung des Gehäuses weist jede der zuletzt genannten vier Gehäusewände eine Fläche auf, die sehr viel kleiner bzw. weniger ausgedehnt als diejenige der zuerst genannten ausgedehnten, größeren bzw. großen Seitenwände ist. Der Abstand zwischen den kleineren bzw. kleinen Seitenwänden definiert die Breite des Reaktorraums, der Abstand zwischen den Stirnwänden definiert die Länge des Reaktorraums, und der Abstand zwischen den größeren Seitenwänden definiert die Dicke bzw. Tiefe oder Höhe des Reaktorraums. Der Eintrag von Licht in den Reaktorraum erfolgt bevorzugt durch eine oder beide der größeren Seitenwände des plattenförmigen Gehäuses.

Es ist möglich, vorzusehen, dass das Gehäuse lediglich teilweise aus einem lichtdurchlässigen Material hergestellt ist, das zumindest einen Teil einer großen Seitenwand oder bevorzugt zumindest eine gesamte solche große Seitenwand bildet. Es ist jedoch bevorzugt, dass auch weitere Teile des Gehäuses und bevorzugt das gesamte oder im wesentlichen das gesamte Gehäuse aus einem lichtdurchlässigen Material ausgebildet ist. Vorteilhafte lichtdurchlässige Materialien sind lichtdurchlässige, durchsichtige oder glasklare Kunststoffmaterialien, wie zum Beispiel PET, mit einer hohen Durchlässigkeit, insbesondere für die photosynthetisch aktive Strahlung zwischen 400 und 700 nm. Es ist bevorzugt, dass die Durchlässigkeit in diesem photosynthetisch wirksamen Spektralbereich mindestens 90% beträgt.

In dem Reaktorraum sind mehrere Zwischenwände oder Leitplatten angeordnet, die bevorzugt ebenfalls aus einem lichtdurchlässigen Material, wie zum Beispiel einem der oben in Zusammenhang mit dem Gehäuse genannten Materialien, ausgebildet sind. Diese Zwischenwände verlaufen parallel zu den kleineren Seitenwänden und parallel zueinander, d.h. sie erstrecken sich entlang der Länge des Reaktorraums und des Gehäuses. Jede Zwischenwand erstreckt sich über die gesamte Dicke des Reaktorraums, so dass sie die beiden größeren Seitenwände miteinander verbindet. Die Zwischenwände sind in der Nähe einer ersten der beiden Stirnwände und, zum Beispiel bevorzugt 2 bis 20 cm und mehr bevorzugt 6 bis 10 cm, beabstandet von dieser ersten Stirnwand angeordnet oder weisen eine Öffnung oder einen Spalt im Bereich der ersten Stirnwand auf. Dabei sind die Zwischenwände bevorzugt alle gleich weit von der ersten Stirnwand beabstandet.

Die Zwischenwände erstrecken sich über einen Teil der Länge des Reaktorraums. Auf diese Weise wird in der Nähe der ersten Stirnwand ein Strömungsleitabschnitt des Reaktorraums mit einer Vielzahl von Strömungsleitkanälen gebildet, von denen sich jeder zwischen zwei benachbarten Zwischenwänden oder zwischen einer der kleineren Seitenwände und einer benachbarten Zwischenwand befindet. Zwischen den der ersten Stirnwand abgewandten Enden der Zwischenwände und der zweiten der beiden Stirnwände ist ein freier bzw. im wesentlichen freier Durchmischungsabschnitt des Reaktorinnenraums vorgesehen, in dem sich keine Zwischenwände befinden. Die Zwischenwände haben bevorzugt identische Längen. Ferner ist es bevorzugt, dass die Zwischenwände gleichmäßig voneinander und von den kleineren Seitenwänden beabstandet sind. Dabei sind die Zwischenwände so angeordnet und dimensioniert, dass sich der Durchmischungsabschnitt des Reaktorraums über mindestens 50%, bevorzugt mindestens 60% und am meisten bevorzugt mindestens 80 % der Länge des Reaktorraums erstreckt und dass die Strömungsleitkanäle eine Breite, d.h. einen Abstand zwischen benachbarten Zwischenwänden bzw. zwischen einer kleinen Seitenwand und der benachbarten Zwischenwand, von mindestens 5 cm aufweisen. Ferner ist es bevorzugt, dass sich die Strömungsleitkanäle über mindestens 10% der Länge des Reaktorraums erstrecken. Es ist ferner bevorzugt, dass die Länge der Strömungsleitkanäle 10 bis 200 cm und mehr bevorzugt 60 bis 100 cm beträgt. In einer vorteilhaften Ausgestaltung sind mindestens fünf und bevorzugt sieben bis neun Strömungsleitkanäle vorgesehen.

In der Nähe der ersten Stirnwand ist eine Gaszufuhreinrichtung zum Einbringen von Gas in jeden zweiten der Strömungsleitkanäle angeordnet.

Im Betrieb wird mit Hilfe der Gaszufuhreinrichtung gleichzeitig Gas in das der ersten Stirnwand zugewandte Ende jedes zweiten der Strömungsleitkanäle eingedüst. Um eine Bewegung des Gases entlang der Strömungsleitkanäle zu erhalten, wird der Bioreaktor dabei so angeordnet, dass die erste Stirnwand tiefer als die zweite Stirnwand liegt und die erste Stirnwand somit im Anwendungsfall die Unterseite des Bioreaktors bildet. Aufgrund der Breite der Strömungsleitkanäle von mindestens 5 cm kann sich in diesen bei hinreichender Begasungsrate und -art eine durch den Gaseintrag induzierte Flüssigkeitsströmung hoher Geschwindigkeit ausbilden. Das Gas steigt in jedem zweiten Strömungsleitkanal nach oben und erzeugt durch den Auftrieb einen Flüssigkeitsstrom nach oben (Airlift). Dabei reißt zudem das Gas Flüssigkeit vom unteren Ende des Reaktorraums mit, und die Flüssigkeitssäulen in den nicht begasten Strömungsleitkanälen drücken aufgrund ihrer größeren Masse die Flüssigkeit in den begasten Strömungsleitkanälen nach oben. In den nicht begasten Strömungsleitkanälen bewirkt der Sog der in den begasten Strömungsleitkanälen aufsteigenden Flüssigkeit, dass sich in den nicht begasten Strömungsleitkanälen eine Flüssigkeitsströmung nach unten ausbildet. Durch diese wird Flüssigkeit aus dem Oberteil des Bioreaktors bis auf dessen Boden in der Nähe der ersten Stirnwand transportiert. In der Realisierung dieses Konvektionsprinzips wird demnach alternierend jeder zweite Strömungsleitkanal begast, unabhängig von der Breite des Bioreaktors und der Anzahl der Strömungsleitkanäle.

Erreicht das in die Strömungsleitkanäle eingedüste Gas das obere Ende der Zwischenwände, und damit das Ende des Strömungsleitabschnitts des Reaktorraums, so steigt das Gas weiter auf und erzeugen zusammen mit den ab- und aufsteigenden Flüssigkeitsströmungen eine stark turbulente dreidimensionale Durchmischung in dem Durchmischungsabschnitt des Reaktorraums und ggf. auch in den Strömungsleitkanälen.

Im Rahmen der vorliegenden Erfindung ist festgestellt worden, dass es durch den gewählten Aufbau überraschenderweise möglich ist, auch außerhalb von Strömungsleitkanälen überall in einem ausgedehnten Durchmischungsbereich, der mehr als die Hälfte der Länge des Reaktorraums einnimmt und aus diesem Grunde für die Lichtausnutzung und Kultivierung bestimmend ist, eine sehr starke turbulente Durchmischung zu erzielen, die zu sehr hohen Frequenzen des Hell-Dunkel-Zyklus der Organismen mit einer Zykluszeit von bevorzugt einigen Millisekunden führt. Dies lässt sich zudem auch mit einer geringen Dicke des Bioreaktors bzw. Reaktorraums von bevorzugt 1,5 bis 5 cm erzielen, so dass in vorteilhafter Weise ein großes Verhältnis von beleuchtbarer Gehäuseseitenfläche zu Reaktorraumvolumen realisiert werden kann. Ferner ist es durch die erzielbare starke Turbulenz und den damit verbundenen schnellen periodischen Wechsel der Organismen zwischen der lichtzugewandten beleuchteten Seite des Bioreaktors und den dunklen Bereichen in der Mitte des Reaktorraums - bzw. in der Nähe der lichtabgewandten Seitenfläche, falls der Bioreaktor nur einseitig belichtet wird - möglich, hohe Zelldichten zu kultivieren. Zudem bewirkt die hohe Turbulenz, dass die Organismen so stark bewegt werden, dass sie immer optimal mit den im Kulturmedium gelösten Nährstoffen und mit Gasen versorgt werden und dass sie sich nicht absetzen können. Die starke Turbulenz verhindert auf diese Weise zusammen mit aufsteigenden Gasblasen auch eine Besiedelung der Reaktoroberflächen (Biofilmbildung bzw. Biofouling), die eine Verminderung des Lichteintritts in den Reaktorraum zur Folge hätte.

Beispielsweise ist der Bioreaktor in einer bevorzugten Ausführungsform durch geeignete Wahl der Breite der Strömungsleitkanäle und der übrigen Dimensionen des Bioreaktors so ausgestaltet, dass durch das beschriebene Einbringen von Gas und das beschriebene Aufsteigen von Gasblasen in den Strömungsleitkanälen im gesamten Durchmischungsbereich oder im wesentlichen im gesamten Durchmischungsbereich aufgrund der erzeugten Turbulenz für die Organismen ein Wechsel zwischen hell und dunkel mit einzelnen Dunkelphasen von 10 ms oder weniger bewirkt werden kann und/oder ein turbulenter Stoffaustauschkoeffizient von mindestens 0,016 m²/s erzielt werden kann. Letzteres führt in vorteilhafter Weise dazu, dass räumliche Konzentrationsunterschiede im Reaktorraum weniger als 2% betragen.

Der Reaktorraum des Bioreaktors der vorliegenden Erfindung ist demnach im Vergleich zum Stand der Technik weitergehend für die Kultivierung von phototrophen Organismen optimiert, wobei sich die Optimierung darauf bezieht, die Organismen mit Licht zu versorgen, die Organismen mit im Kulturmedium gelösten Nährstoffen (Stickstoff, Phosphor und Spurenelemente) und Gasen zu versorgen, zu verhindern, dass sich die Organismen absetzen bzw. sedimentieren, und zu verhindern, dass sich Organismen an den Reaktorwänden anheften, wodurch stets ein ungehinderter Lichteintritt in den Reaktorraum gewährleistet ist.

Ferner lässt sich ein im Vergleich zu anderen Reaktortypen günstigeres Verhältnis von Oberfläche und Volumen verwirklichen, d.h. die plattenförmigen Bioreaktoren können sehr dünn und deshalb für geringe Volumina an Kulturmedium ausgelegt werden. So weist der Reaktorraum in einer bevorzugten Ausgestaltung des Bioreaktors eine Dicke von 1,5 bis 5 cm auf, mehr bevorzugt von 2,0 bis 3,0 cm. Die Länge des Reaktorraums beträgt bevorzugt 100 bis 600 cm, mehr bevorzugt 300 bis 400 cm, und die Breite des Reaktorraums beträgt bevorzugt 30 bis 400 cm, mehr bevorzugt 100 bis 200 cm. In jedem Fall ist es vorteilhaft, wenn die Länge des Reaktorraums größer als seine Breite ist.

Durch das günstige Verhältnis von Oberfläche und Volumen ist es in vorteilhafter Weise möglich, den Einsatz an Ressourcen zur Kultivierung (u.a. Wasser, Nährstoffe, Gas und Energie) zu verringern. Im Betrieb werden bevorzugt Zelldichten im Reaktorraum eingestellt, die eine optische Dichte von 7 oder ungefähr 7 bewirken, was einem Hell-Dunkel-Aufenthaltszeitverhältnis von etwa 1:2 bis 1:3 entspricht. Diese Einstellung kann über die Zelldichte als Solltrockenmasse in g/l erfolgen. Diese beträgt beispielsweise bei einer Dicke des Bioreaktors von 5 cm bevorzugt 0,9 g/l, bei einer Dicke von 3 cm bevorzugt 1,5 g/l und bei einer Dicke von 1,5 cm bevorzugt 3 g/l. Bis zu diesen optisch begründeten Solltrockenmassen findet keine Unterversorgung der Organismen mit Licht statt, so dass alle Organismen gleichmäßig photosynthetisch aktiv gehalten werden können.

Erfindungsgemäße Bioreaktoren mit Flächen von mehr als einem Quadratmeter großer Seitenwand lassen sich zum Beispiel in vorteilhafter Weise in großtechnischem Maßstab zur Kultivierung von Algen einsetzen, um CO₂ aus Rauchgas zu binden und aus der dabei erzeugten Biomasse wirtschaftlich Energie zu gewinnen. Dabei ist die Möglichkeit von besonderem Vorteil, den Bioreaktor mit geringem Energie- und Ressourceneinsatz zu betreiben und eine hohe Produktion von Biomasse pro Reaktorfläche zu erzielen.

In einer vorteilhaften Ausgestaltung des Bioreaktors beträgt die Breite der Strömungsleitkanäle mindestens 10 cm und bevorzugt 10 bis 50 cm. In einer weiteren vorteilhaften Ausgestaltung beträgt die Breite der Strömungsleitkanäle mindestens 15 cm und bevorzugt 15 bis 20 cm.

Um das unerwünschte Absetzen von Organismen an den Reaktorraumwänden weiter zu erschweren, kann es vorteilhaft sein, einzelne oder alle der Ecken und/oder Kanten des Bioreaktorgehäuses abgerundet auszubilden.

In einer bevorzugten Ausführungsform des Bioreaktors sind in den Zwischenwänden entlang ihrer Länge jeweils eine Anzahl voneinander beabstandeter Öffnungen bzw. Durchgangsbohrungen vorgesehen, durch die ein Flüssigkeitsaustausch zwischen benachbarten Strömungsleitkanälen stattfinden kann. Durch diese Ausgestaltung wird bewirkt, dass das in jedem zweiten Strömungsleitkanal aufsteigende Gas bei der hydrostatischen Erzeugung einer Flüssigkeitsströmung nach oben nicht nur Flüssigkeit vom unteren Ende des Reaktorraums, d.h. aus der Nähe der ersten Stirnwand, mitreißt, sondern auch zusätzlich über die Öffnungen aus den angrenzenden, nicht begasten Strömungsleitkanälen. Dadurch wird erreicht, dass die Gasblasen in den begasten Strömungskanälen über den gesamten Strömungskanal ungehindert aufsteigen können, hohe Geschwindigkeiten erreichen und somit hohe Strömungsgeschwindigkeiten erzielt werden können. Darüber hinaus entstehen durch das seitliche Einströmen von Flüssigkeit in die begasten Strömungskanäle in den Strömungsleitkanälen Verwirbelungen, so dass nicht nur im Durchmischungsbereich, sondern auch im Bereich der Strömungsleitkanäle turbulente Durchmischungen hoher Geschwindigkeit stattfinden können. Es ist besonders bevorzugt, wenn die Öffnungen bzw. Durchgangsbohrungen in Bereichen der Zwischenwände vorgesehen sind, die an eine der großen Seitenwände des Gehäuses angrenzen, und zwar an die große Seitenwand, die im Betrieb nicht dazu vorgesehen ist, von Licht durchstrahlt zu werden, d.h. die große Seitenwand, die bei einer geneigten Anordnung des Bioreaktors der sonnenabgewandten Seite entspricht. Dadurch wird verhindert, dass Teile der aufsteigenden großen Gasblasen ganz oder teilweise in diejenigen Strömungsleitkanäle gelangen, in denen die Flüssigkeit nach unten strömt, dort aufsteigen und so die Flüssigkeitsströmung abbremsen. Die Öffnungen bzw. Durchgangsbohrungen können in einer vorteilhaften Ausgestaltung Längen in Richtung des Verlaufs der Strömungsleitkanäle von 1 bis 30 cm und mehr bevorzugt 3 bis 5 cm und Abmessungen senkrecht zum Verlauf der Strömungsleitkanäle von 10 bis 90% und mehr bevorzugt von 50% oder etwa 50% der Höhe der Zwischenwände aufweisen.

Es ist bevorzugt, dass die Gaszufuhreinrichtung ein Rohr oder mehrere Rohre aufweist, das bzw. die in dem Reaktorraum zwischen der ersten Stirnwand und den dieser zugewandten Enden der Zwischenwände verläuft bzw. verlaufen und Bohrungen oder Schlitze aufweist bzw. aufweisen, durch die Gas in die jeweiligen zu begasenden Strömungsleitkanäle abgegeben wird. Dabei ist es ferner bevorzugt, dass das Rohr bzw. die Rohre parallel zu der ersten Stirnwand verläuft bzw. verlaufen. Die Dicke des Rohrs bzw. der Rohre sollte bevorzugt nicht mehr als ein Drittel der Dicke des Reaktorraums betragen, um die Strömung nicht zu stark zu behindern. Aus demselben Grunde ist es vorteilhaft, wenn das Rohr bzw. die Rohre in einem gewissen Abstand zu den der ersten Stirnwand zugewandten Enden der Zwischenwände angeordnet sind. Zum Beispiel ist es bevorzugt, dass das Rohr bzw. die Rohre in einem Abstand von 5 bis 150 mm und mehr bevorzugt von 30 bis 50 mm von der ersten Stirnwand angeordnet sind. Ferner ist es bevorzugt, dass das Rohr bzw. die Rohre in einem Abstand von 5 bis 150 mm und mehr bevorzugt von 30 bis 50 mm von den der ersten Stirnwand zugewandten Enden der Zwischenwände angeordnet sind. Das Rohr sollte ferner auch deshalb einen möglichst geringen Innendurchmesser aufweisen, weil bei einer stossweisen Begasung Flüssigkeit ins Rohr eintritt und mit dem nächsten Stoß zunächst erst wieder ausgeblasen werden muß. Bei dünnen Rohren kommt es daher zu weniger Verzögerungszeit. Beispielsweise beträgt der Innendurchmesser des Begasungsrohres für eine Reaktorbreite von 1 m bevorzugt 5 mm.

In Zusammenhang mit einer solchen Gaszufuhreinrichtung ist es ferner vorteilhaft, wenn die Gaszufuhreinrichtung angepasst ist, um, bevorzugt automatisch, beispielsweise mit Hilfe einer geeigneten Steuereinrichtung, die Teil des Bioreaktors sein kann, bei mit flüssigem Kulturmedium und phototrophen Organismen befülltem Reaktorraum Gas in Form einzelner, zusammenhängender Gasblasen in die jeweiligen Strömungsleitkanäle einzubringen. Die Gasblasen weisen dabei einen großen Durchmesser (d.h. der maximale Durchmesser einer gegebenen Gasblase) von mindestens 3 cm, bevorzugt von 3 bis 50 cm und mehr bevorzugt von 10 bis 15 cm auf. Durch den Aufstieg derartiger einzelner, großer Gasblasen in den Strömungsleitkanälen wird eine hohe Geschwindigkeit des Gasaufstiegs und infolgedessen eine hohe Sogwirkung erreicht, was für die im Durchmischungsabschnitt zu erzielende starke Turbulenz günstige hohe Strömungsgeschwindigkeiten mit sich bringt, die in den Strömungsleitkanälen mindestens so groß wie die Gasblasengeschwindigkeit ist. Es ist besonders bevorzugt, wenn die Gasblasen einen großen Durchmesser aufweisen, der der Breite des jeweiligen Strömungsleitkanals entspricht oder größer ist. Eine solche Blase wirkt wie ein Saugheber bzw. Kolben und entwickelt somit die größte Sogwirkung. Die Abmessungen der Schlitze bzw. die Größe der Bohrungen in dem Rohr bzw. den Rohren richtet sich im allgemeinen nach der Menge des einzublasenden Gases bzw. dem Druck mit dem es eingeblasen wird und sollen bevorzugt groß genug sein, damit in einem Zeitraum von 100 bis 1000 ms die oben genannten Blasengrößen erreicht werden. Bevorzugt haben die Bohrungen einen Durchmesser von 0,5 bis 4 mm und mehr bevorzugt 1 bis 2 mm, und die Schlitze haben bevorzugt eine Breite von 0,2 bis 1 mm und mehr bevorzugt von 0,4 bis 0,6 mm. Es ist dabei ferner bevorzugt, dass die Gaszufuhreinrichtung angepasst ist, um das Gas so einzubringen, dass die Gasblasengeschwindigkeit in den Strömungsleitkanälen mindestens 0,5 m/s beträgt. Die Größe und Geschwindigkeit der Gasblasen wird allgemein so gewählt, dass die oben angegebenen vorteilhaften Strömungs- und Turbulenzcharakteristika entstehen. Insbesondere ist es bevorzugt, wenn die Größe und die Geschwindigkeit der Gasblasen in der Weise auf die Ausgestaltung des Bioreaktors abgestimmt sind, dass in der gesamten oder im wesentlichen in der gesamten Flüssigkeit im Reaktorraum überkritische Reynoldszahlen vorliegen, so dass in der gesamten oder im wesentlichen in der gesamten Flüssigkeit turbulente Strömungsbedingungen herrschen.

Um den Antrieb mit möglichst wenig Gas zu erreichen, ist es ferner bevorzugt, wenn die Gaszufuhreinrichtung angepasst ist, um das Gas, bevorzugt automatisch, beispielsweise mit Hilfe einer geeigneten Steuereinrichtung, die Teil des Bioreaktors sein kann, in der Weise stoßweise in die entsprechenden Strömungsleitkanäle einzubringen, dass erst dann eine neue Gasblase erzeugt wird, wenn die vorhergehende Gasblase das Kulturmedium in dem Reaktorraum vollständig durchlaufen hat. In einer bevorzugten Ausführungsform beträgt die Impulslänge des stoßweisen Einbringens von Gas 100 bis 1500 ms mit einer Periodizität von 500 bis 10000 ms und mehr bevorzugt 400 bis 600 ms mit einer Periodizität von 1500 bis 4000 ms.

Insgesamt kann im Rahmen der vorliegenden Erfindung zur Bewirkung der Flüssigkeitsströmung in vorteilhafter Weise mit geringen Gasdrücken von bevorzugt 0,1 bis 2,0 bar Überdruck und mehr bevorzugt 0,5 bis 1,0 bar Überdruck gearbeitet werden.

In einer bevorzugten Ausführungsform sind in dem Durchmischungsabschnitt des Reaktorraums eine Vielzahl voneinander beabstandeter Säulen oder Stäbe angeordnet, die jeweils die beiden großen Seitenwände des Gehäuses miteinander verbinden und bevorzugt jeweils so ausgebildet sind, dass sie sich zumindest in Richtung auf die große Seitenwand, z.B. konisch, verjüngen, die der vorgesehenen Lichteinstrahlung abgewandt ist. Durch diese Ausgestaltung wird in vorteilhafter Weise die mechanische Festigkeit gegenüber Vibrationen und Schwingungen der Gehäusewände und der Zwischenwände erhöht, die im Betrieb aufgrund des Flüssigkeitsdrucks auftreten können. Allgemein dienen derartige Säulen oder Stäbe dazu, die Gehäusekonstruktion mechanisch zu stabilisieren. Diese Stabilisierungsfunktion wird im Strömungsleitabschnitt des Reaktorraums von den Zwischenwänden übernommen. Außerdem wirken die Säulen oder Stäbe wie statische Mischer an denen sich die Flüssigkeitsströmungen brechen, was die turbulente Durchmischung weiter erhöht. Die Säulen bzw. Stäbe können maximale Querschnittsdurchmesser von 1 bis 10 cm und mehr bevorzugt von 3 bis 5 cm aufweisen.

Ein derart aufgebauter Bioreaktor kann in vorteilhafter Weise aus zwei Halbschalen gefertigt werden, die zum Beispiel durch Tiefziehen hergestellt und anschließend miteinander verbunden werden, oder in einem Stück durch Twin Sheet oder beispielsweise Spritzgieß- oder Rotationsverfahren.

In einer vorteilhaften Ausführungsform ist der Bioreaktor an einer Haltevorrichtung befestigt, mit deren Hilfe der Neigungswinkel des Bioreaktors in Bezug auf den Boden bzw. die Stellfläche und ggf. auch die Winkelposition in Bezug auf eine senkrecht zur Erdoberfläche verlaufende Achse verändert werden kann. Da der Bioreaktor mit Neigungswinkeln zwischen nahezu 0° und 90° und bevorzugt zwischen 30° und 90° betrieben werden kann, ist es besonders vorteilhaft, wenn die Neigungswinkelvariation diskret oder kontinuierlich in diesen Bereichen möglich ist. Auf diese Weise ist es möglich, den Bioreaktor im Tagesverlauf nach dem Sonnenstand derart auszurichten, dass das Sonnenlicht zu jedem Zeitpunkt in einem optimalen Winkel in den Reaktorraum einstrahlt. Ferner ist es möglich, den Bioreaktor in einfacher Weise in eine gegen Schäden durch Sturm geschützte Stellung und bei zu hoher Sonneneinstrahlung in eine Schattenstellung zu bringen. Als Haltevorrichtung können zum Beispiel geeignete, aus der Solartechnik bekannte Nachführsysteme zum Einsatz kommen.

Die Bioreaktoren können einzeln oder miteinander verbunden als Batch oder als Turbidostat betrieben werden. Als Batch findet ein Austausch des Kulturmediums in bestimmten Zeitabschnitten statt. Als Turbidostat wird das Kulturmedium kontinuierlich über die Zeit ausgetauscht bzw. im Kreislauf zurückgeführt, d.h. es findet ein kontinuierlicher Zu- und Ablauf statt. Um diesen Betrieb zu ermöglichen, weist der Bioreaktor in einer vorteilhaften Ausführungsform einen Zulaufanschluss auf, über den die Flüssigkeit mit Hilfe einer Pumpe in den Reaktorraum gepumpt werden kann, und einen Ablaufanschluss, über den die Flüssigkeit abgegeben wird. Der Ablaufanschluss kann bevorzugt am oberen Ende des Bioreaktors, d.h. an der zweiten Stirnwand vorgesehen sein, und der Ablauf kann bevorzugt durch diesen Ablaufanschluss zusammen mit der Abgabe von Gas aus dem Reaktorraum stattfinden. Nach Trennung des Gas-Wasser-Gemisches kann die Flüssigkeit dann alleine über den hydrostatischen Druck bis zur Pumpe geführt werden. Dabei bzw. an anderer Stelle im äußeren Kreislaufabschnitt kann in vorteilhafter Weise durch geeignete Einrichtungen sowohl die Aufnahme von Messparametern als auch die Zudosierung von Nährstoffen und ggf. von Säuren oder Basen zur Einstellung des pH-Wertes im Kulturmedium erfolgen. Darüber hinaus können über die Kreislaufführung Teilströme des Kulturmediums erwärmt oder abgekühlt werden, um optimale Temperaturbedingungen in dem Bioreaktor zu erreichen. Eine Erwärmung ist bevorzugt durchzuführen, wenn die Temperatur im Bioreaktor unter 10 °C sinkt, wobei im Teilstrom Temperaturen bis zu 30 °C eingesetzt werden können. Es ist bevorzugt, wenn der Volumenstrom des Kulturmediums bei Kreislaufführung in Prozent des Bioreaktorvolumens pro Stunde 0 bis 200% und mehr bevorzugt 50 bis 100% beträgt. Dabei bedeutet ein Wert von 50% zum Beispiel, dass das Bioreaktorvolumen alle zwei Stunden einmal umgewälzt wird.

Es ist ferner für das Wachstum der phototrophen Organismen von Vorteil, wenn zusätzlich zu dem beschriebenen, zum Bewirken der Turbulenz dienenden Gaseintrag auch unabhängig CO₂ in das Kulturmedium eingebracht wird. Zu diesem Zweck können Diffusoren und/oder statische Mischer vorgesehen sein. Die Diffusoren können in vorteilhafter Weise als poröse Schläuche oder Rohre mit einer Porengröße von bevorzugt 5 bis 35 µm ausgebildet sein, wobei für die Anordnung und Dimensionierung dasselbe wie für die Rohre zum Einbringen des Gases zum Antrieb der Flüssigkeitsbewegung gilt. Die Diffusoren sind bevorzugt im Bereich zwischen der ersten Stirnwand und den dieser zugewandten Enden der Zwischenwände im Reaktorraum angeordnet und weisen einen, beispielsweise durch eine der größeren Seitenwände vorgesehenen Zulauf auf, über den die Beaufschlagung mit Gas erfolgt. Das Gas tritt dann im Betrieb über die Poren als feinste Gasblasen ins Kulturmedium über. Da die Geschwindigkeit des Gasaustausches zwischen Gas und Flüssigkeit von der Größe bzw. der Oberfläche der Gasblasen abhängt, ist dieser umso größer, je kleiner die Gasblasen sind. Die Größe der Poren ist also so zu wählen, dass möglichst kleine Gasblasen entstehen. Bekannte statische Mischer können genutzt werden, um das Gas in einem Rohrleitungssystem im Kulturmedium zu lösen. So ist es zum Beispiel im Fall des Betriebs der Bioreaktoren als Turbidostaten möglich, das im Kreislauf geführte Kulturmedium über statische Mischer mit Gas anzureichern. Der Gasdruck in den Diffusoren kann in vorteilhafter Weise relativ niedrig gewählt werden. Bevorzugte Werte sind 0,1 bis 2 bar Überdruck, mehr bevorzugt 0,4 bis 0,6 bar Überdruck.

In einer vorteilhaften Ausgestaltung ist die Oberfläche der Bioreaktoren - und bevorzugt eine oder beide der großen Seitenwände - vollständig oder zumindest teilweise mit Dünnfilmsolarzellen beschichtet.

Die oben beschriebenen Bioreaktoren können in vorteilhafter Weise zur Kultivierung phototropher Zellen und/oder Organismen eingesetzt werden. Unter phototrophen Organismen werden vorliegend insbesondere Mikroalgen verstanden. Mikroalgen umfassen wie vorliegend verwendet einzellige oder mehrzellige Organismen aus der Gruppe der eukaryotischen Algen oder aus der Gruppe der prokaryontischen Cyanobakterien, die zwischen 1 µm und 100 µm groß sind. Als Algen werden eukaryontische Organismen verstanden, die zu den Pflanzen zählen. Algen, und insbesondere Mikroalgen, werden industriell für die Produktion verschiedener biologischer Substanzen, wie beispielsweise Polysaccharide, Fettsäuren, Proteine, Lipide, Farbstoffe, Vitamine und Sterole, verwendet. Für die Produktion von Fettsäuren und Ölen werden bevorzugt Mikroalgen verwendet, die Chlorophyll a und c besitzen und Lipide statt Stärke als Speicherstoffe aufbauen. Solche Mikroalgen sind bevorzugt in den taxonomischen Gruppen der Heterokontophyta, Dinophyceae, Cyanophyceen, Haptophyta zu finden. Biomassen aus Mikroalgen findet darüber hinaus Anwendung als Futterstoff. Vorzugsweise handelt es sich bei den Mikroalgen, die mit Hilfe der erfindungsgemäßen Verfahren und Bioreaktoren kultiviert werden sollen, um solche der Gattungen Phaeodactylum, Isochrysis, Monodus, Porphyridium, Spirulina, Chlorella, Botryococcus, Cyclotella, Nitzschia, Dunaliella und/oder Nannochloropsis.

Bei den Cyanobakterien handelt es sich um prokaryotische Organismen, die in der Lage sind, oxygene Photosynthese zu betreiben. Cyanobakterien weisen neben Chlorophyll weitere lichtadsorbierenden Pigmente aus der Gruppe der Phycobiline, die ihnen eine im Vergleich zu Pflanzen optimalere Lichtnutzung ermöglichen. Aus diesem Grund können Cyanobakterien Schwachlichtbereiche besiedeln, in die Pflanzen aufgrund der auf Chlorophyll basierenden Photosynthesesysteme nicht vordringen können. Die vorliegenden Bioreaktoren und Verfahren können prinzipiell mit beliebigen Arten von Cyanobakterien betrieben werden. Vorzugsweise handelt es sich bei den zu kultivierenden Cyanobakterien um solche der Gattungen, Acaryochloris, Anabaena, Aphanizomenon, Chroococcus, Gloeobacter, Gloeocapsa, Lyngbya, Microcystis, Microcoleus, Nodularia, Nostoc, Oscillatoria, Phormidium, Prochlorococcus, Prochloron, Prochlorothrix, Scytonema, Spirulina, Stigonema, Synechococcus und/oder Trichodesmium.

Ferner lassen sich auch pflanzliche Zellkulturen und Moose in den Bioreaktoren anzüchten.

Der beschriebene Bioreaktor kann vorteilhaft im Rahmen eines Verfahrens zur Kultivierung der oben genannten phototrophen Organismen verwendet werden, bei dem ein flüssiges Kulturmedium und photothrophe Organismen in den Reaktorraum des Bioreaktors eingebracht werden und eine der beiden größeren Seitenwände des Gehäuses des Bioreaktors, die teilweise oder bevorzugt vollständig aus lichtdurchlässigem Material ausgebildet ist, oder beide dieser Seitenwände derart mit Licht bestrahlt wird, dass das Licht in den Reaktorraum gelangt. Gleichzeitig wird in jeden zweiten Strömungsleitkanal mit Hilfe der Gaszufuhreinrichtung Gas eingedüst. Dieses Eindüsen erfolgt in der Weise, dass das Gas, wie oben bereits beschrieben wurde, in Form einzelner Blasen mit einem großen Durchmesser von mindestens 3 cm in den entsprechenden Strömungsleitkanälen aufsteigt. Es hat sich gezeigt, dass es auf diese Weise aufgrund der erfindungsgemäßen Ausgestaltung des Bioreaktors möglich ist, in der oben beschriebenen Weise eine ausreichend starke dreidimensionale Turbulenz mit den bereits angegebenen Eigenschaften nicht nur in den Strömungskanälen, sondern auch im gesamten ausgedehnten Durchmischungsbereich zu erreichen, verbunden mit der oben beschriebenen vorteilhaften Bewegung der phototrophen Organismen.

In einer bevorzugten Ausgestaltung wird das Gas mit Hilfe der Gaszufuhreinrichtung so eingedüst, dass im gesamten Durchmischungsbereich oder im wesentlichen im gesamten Durchmischungsbereich aufgrund der erzeugten Turbulenz ein Wechsel der Organismen zwischen hell und dunkel mit einzelnen Dunkelphasen von 10 ms oder weniger bewirkt wird und/oder ein turbulenter Stoffaustauschkoeffizient von mindestens 0,016 m²/s erzielt wird. Letzteres führt in vorteilhafter Weise dazu, dass räumliche Konzentrationsunterschiede im Reaktorraum weniger als 2% betragen.

Die Größe und Geschwindigkeit der Gasblasen wird so gewählt, dass die gewünschten vorteilhaften Strömungs- und Turbulenzcharakteristika entstehen, bevorzugt mit überkritischen Reynoldszahlen in der gesamten oder im wesentlichen in der gesamten Flüssigkeit im Reaktorraum. Es ist bevorzugt, dass das Gas mit Hilfe der Gaszufuhreinrichtung so eingebracht wird, dass die Geschwindigkeit der das Kulturmedium in den Strömungsleitkanälen durchlaufenden Gasblasen mindestens 0,5 m/sec beträgt.

Es ist ferner bevorzugt, dass das zum Antreiben der Flüssigkeitsströmung verwendete Gas mit einem Volumenstrom pro Fläche der Stirnwand, über die das Gas eingebracht wird, (sog. Filtergeschwindigkeit) von mindestens 0,02 m/sec in die begasten Strömungskanäle eingebracht wird.

Außerdem ist es bevorzugt, dass das Gas in der Weise stoßweise, und bevorzugt periodisch, in die entsprechenden Strömungsleitkanäle eingebracht wird, dass erst dann eine neue Gasblase erzeugt wird, wenn die vorhergehende Gasblase das Kulturmedium in dem Reaktorraum vollständig durchlaufen hat. Auf diese Weise kann die angestrebte stark turbulente Bewegung mit möglichst wenig Gas erreicht werden. Die Länge der Periode, mit der der Gaseintrag erfolgt, richtet sich nach der Zeit, die eine Gasblase braucht, um bis zur Oberkante des Reaktors bzw. bis zur der zweiten Stirnwand des Gehäuses benachbarten Flüssigkeitsoberfläche des Kulturmediums zu gelangen. Die Länge des Gasstoßes richtet sich nach der Größe der dabei zu erzeugenden Gasblase.

Der Aufbau des Bioreaktors sowie die Kreislaufführung als Turbidostat ermöglichen ein hohes Maß an Automatisierung der Prozess- bzw. Anlagenführung. Insbesondere ist es in vorteilhafter Weise möglich, über die Größe und Periodizität des Gaseintrags in den Reaktorraum die Strömungsgeschwindigkeit und dementsprechend die Periodizität des Hell-Dunkel-Zyklus einzustellen bzw. zu variieren. Das Lichtklima im Reaktorraum kann über die Zelldichte sowie über die Variation des Neigungswinkels bzw. die Ausrichtung des Reaktors zur Sonne im Tages- und Jahresverlauf eingestellt werden. Bei einer Kreislaufführung können sämtliche Wasserinhaltsstoffe kontrolliert bzw. variiert werden, und es kann in einfacher Weise eine Ernte der Organismen erfolgen.

Es ist vorteilhaft, wenn das beschriebene Verfahren mit einem Bioreaktor oder mit mehreren, miteinander verbundenen Bioreaktoren durchgeführt wird, wobei Teilströme an Kulturmedium gleichzeitig mit der Zufuhr von neuem oder zurück geführtem Kulturmedium kontinuierlich aus dem Reaktor ausgeschleust werden. Im Verlaufe des Verfahrens werden Nährstoffe, Wäre, Substanzen zur Einstellung des pH-Wertes und Gase in den Reaktorraum bzw. in die Reaktorräume eingebracht und phototrophe Organismen geerntet. Nährstoffe können einzeln oder als Mischung in wässriger Form zudosiert werden, sobald sie unter einen Grenzwert absinken, der in geeigneter Weise messtechnisch überwacht werden kann. Als Nährstoffe können sowohl mineralische als auch organische Dünger verwendet werden. Als organischer Dünger ist Gülle aus der Masttierhaltung vorteilhaft. Wärme kann in vorteilhafter Weise dann über einen Wärmetauscher in das Kulturmedium eingebracht werden, wenn die Temperatur des Kulturmediums im Reaktor 10 °C unterschreitet, und zwar in der Weise, dass die Temperatur auf 12 bis 30 °C gebracht wird. Basen, Säuren oder Puffer werden bevorzugt dann hinzudosiert, wenn ein Grenzwert für pH unterschritten wird, der in geeigneter Weise messtechnisch überwacht werden kann. Die Gase werden in einer vorteilhaften Ausgestaltung über einen statischen Mischer bis zur Sättigung in das Kulturmedium eingebracht. Die phototrophen Organismen werden bevorzugt bei Überschreitung eines Grenzwertes für die Zellzahl bzw. der damit korrelierenden Trübung aus dem Kulturmedium abgetrennt.

Die Erfindung wird im Folgenden anhand eines bevorzugten Ausführungsbeispiels unter Bezugnahme auf die beigefügten Zeichnungen beschrieben.
- Figur 1: zeigt einen Querschnitt einer bevorzugten Ausfüh- rungsform des erfindungsgemäßen Bioreaktors.
- Figur 2: zeigt einen Querschnitt des Bioreaktors der Figur 1 in einer Ebene senkrecht zu der des Querschnitts der Figur 1.
- Figur 3: zeigt eine schematische Darstellung der Strömungsver- hältnisse in dem Bioreaktor der Figuren 1 und 2 wäh- rend seines Betriebs.

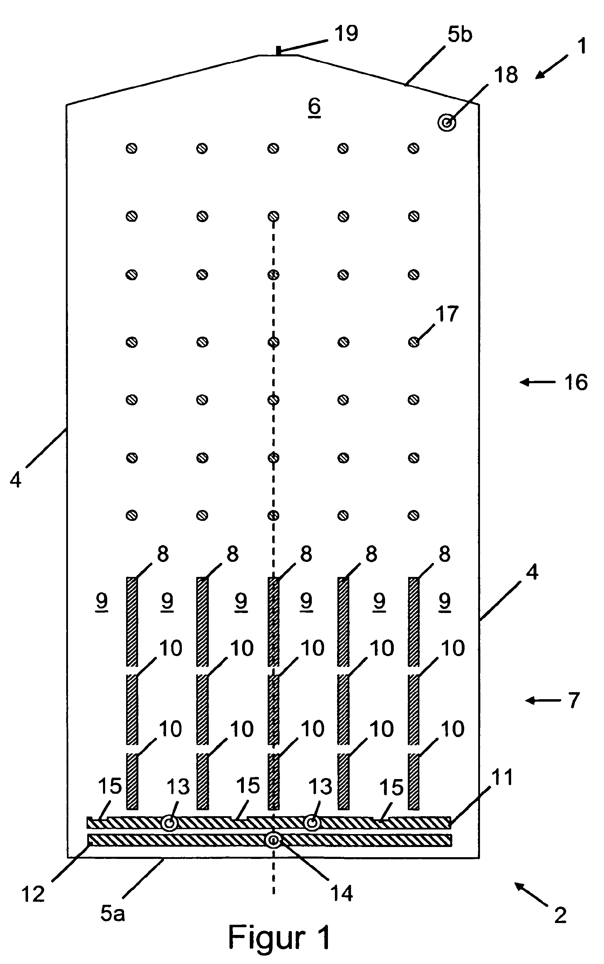

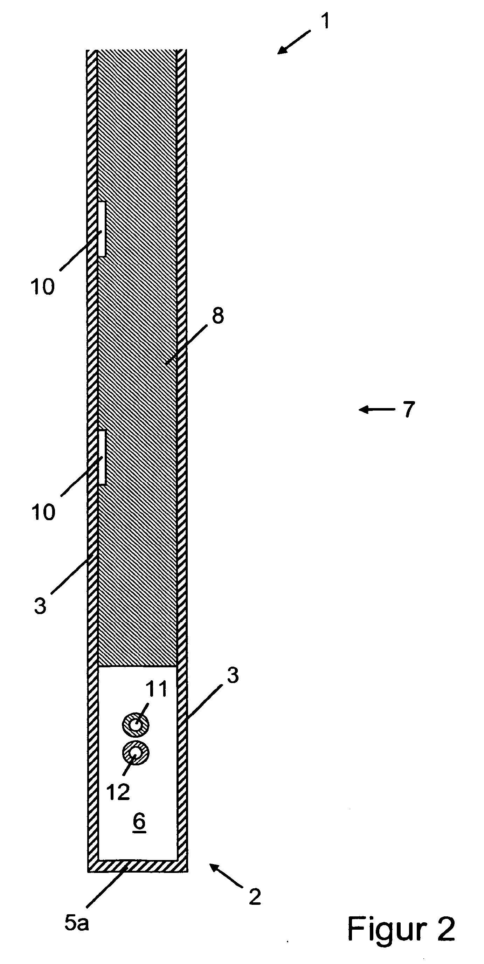

In Figur 1 ist ein plattenförmiger Bioreaktor 1 in einer Querschnittsansicht gezeigt, wobei die Schnittebene entlang der Plattenausdehnung verläuft. Figur 2 zeigt einen Querschnitt eines unteren Bereichs desselben Bioreaktors 1 entlang der Linie II-II in Figur 1. Wie aus den Figuren ersichtlich ist, weist der Bioreaktor 1 ein Gehäuse 2 auf, das zwei einander gegenüberliegende Seitenwände 3, zwei einander gegenüberliegende Seitenwände 4 und zwei einander gegenüberliegende Stirnwände 5a und 5b aufweist, die jeweils aus einem lichtdurchlässigen Material ausgebildet sind. Dabei bilden die Seitenwände 3 die ausgedehnten Seitenflächen des plattenförmigen Gehäuses 2, d.h. die Fläche dieser Seitenwände 3 ist sehr viel größer als die Fläche der restlichen Wände 4, 5a und 5b, so dass das Gehäuse eine flache Gestalt hat. Im Rahmen dieser Anmeldung bestimmt die in Figur 1 sichtbare Ausdehnung der Seitenwände 4 die Länge des Gehäuses 2 und des Reaktorraums 6, die in Figur 1 sichtbare Ausdehnung der Stirnwände 5a, 5b bestimmt die Breite des Gehäuses 2 und des Reaktorraums 6, und die in Figur 2 sichtbare Ausdehnung der Seitenwände 4 und der Stirnwand 5a bestimmt die Höhe bzw. Dicke des Gehäuses 2 und des Reaktorraums 6. Im Betrieb wird der Bioreaktor 1 so angeordnet, dass die Stirnwand 5a die Unterseite des Bioreaktors 1 und die Stirnwand 5b die Oberseite des Bioreaktors 1 bildet, wobei der Neigungswinkel des Gehäuses 2 aber zwischen nahezu 0° und 90° variieren kann.

Die Wände 3, 4, 5a und 5b umgrenzen einen in dem Gehäuse 2 vorgesehenen Reaktorraum 6, der dementsprechend ebenfalls eine flache Ausgestaltung hat. In dem Reaktorraum 6 sind in einem unteren Abschnitt 7, beabstandet von der Stirnwand 5a in regelmäßigen Abständen fünf identische Zwischenwände 8 angeordnet, die parallel zueinander und zu den Seitenwänden 4 verlaufen. Wie aus Figur 2 ersichtlich ist, erstreckt sich jede Zwischenwand 8 zwischen den gegenüberliegenden ausgedehnten bzw. großen Seitenwänden 3, so dass der Reaktorraum 6 im Bereich der Zwischenwände 8 in sechs einzelne Kammern oder Kanäle 9 unterteilt ist. Der Abstand benachbarter Zwischenwände 8 und der Abstand der äußersten Zwischenwände 8 von der benachbarten Seitenwand 4 beträgt jeweils mindestens 5 cm. In jeder Zwischenwand 8 sind dabei bevorzugt entlang ihrer Länge mehrere Öffnungen oder Durchgangsbohrungen 10 ausgebildet, über die benachbarte Kanäle 9 miteinander in Verbindung stehen. Die Öffnungen 9 sind bevorzugt in einem Bereich der Zwischenwände 8 ausgebildet, der an eine der ausgedehnten Seitenwände 3 angrenzt.

In dem Zwischenraum zwischen der Stirnwand 5a und den dieser zugewandten Enden der Zwischenwände 8 sind innerhalb des Reaktorraums 6 zwei beidseitig geschlossene Schläuche oder Rohre 11 und 12 befestigt, die jeweils mit zwei bzw. einem durch eine Seitenwand 3 nach außen führenden Anschlüssen 13 bzw. Anschluss 14 versehen sind, über die bzw. den Gas in die Schläuche oder Rohre 11 und 12 eingebracht werden kann. Das Vorsehen von zwei Anschlüssen 13 hat insbesondere bei niedrigen Drücken den Vorteil, dass im gesamten Schlauch bzw. Rohr gleichmäßige Druckverhältnisse herrschen.

In dem Schlauch oder Rohr 11 ist unterhalb jedes zweiten Kanals 9 ein Schlitz ausgebildet, durch den Gas aus dem Schlauch oder Rohr 11 in den jeweiligen Kanal 9 eintreten kann. Die Schlitze können beispielsweise ein Länge von 20 mm und eine Breite von 0,5 mm haben. Alternativ zu Schlitzen 15 können auch jeweils zwei dicht nebeneinander angeordnete Bohrungen mit jeweils zum Beispiel 2 mm Durchmesser vorgesehen werden. Bei einer typischen Dicke des Reaktorraums 6, d.h. einem Abstand der Innenflächen der Seitenwände 3, von 1,5 bis 5 cm sollte der Durchmesser des Schlauchs oder Rohrs 11 nicht mehr als ein Drittel der Dicke des Reaktorraums 6 betragen und könnte zum Beispiel einen Außendurchmesser von 6 mm und einen Innendurchmesser von 1,5 mm aufweisen.

Der Schlauch oder das Rohr 12 ist porös ausgebildet, so dass beim Einpumpen von Gas durch den Anschluss 14 Gas in Form feiner Gasblasen aus dem Schlauch oder Rohr 12 austritt.

In dem Abschnitt 16 des Reaktorraums 6, der sich von der Stirnwand 5b bis zu den der Stirnwand 5a gegenüberliegenden Enden der Zwischenwände 8 bis Kanäle 9 erstreckt, sind eine Anzahl von säulenförmigen Stützen 17 angeordnet, die jeweils die beiden gegenüberliegenden Seitenwände 4 miteinander verbinden und gegeneinander abstützen. Diese Stützen 17 dienen zusammen mit den Zwischenwänden 8 der mechanischen Stabilisierung des Gehäuses 2.

In einer der Seitenwände 3 ist ferner ein Anschluss 18 vorgesehen, durch den Flüssigkeit in den Reaktorraum 6 eingebracht werden kann, und in der Stirnwand 5b ist ein Anschluss 19 vorgesehen, durch den Gas und Flüssigkeit aus dem Reaktorraum 6 ausgetragen werden kann.

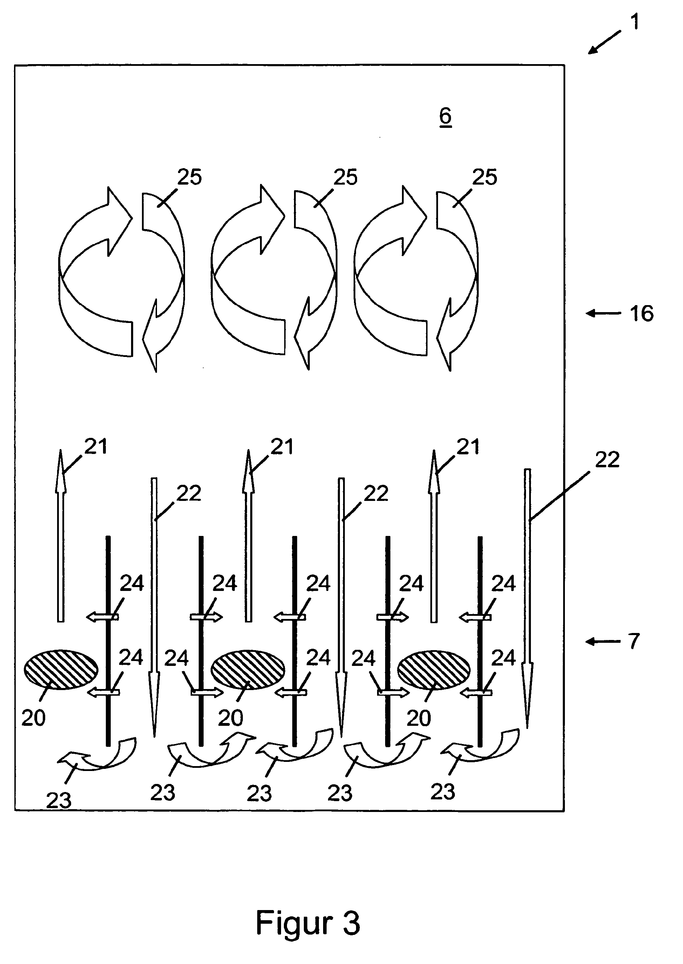

Im Betrieb wird der Reaktorraum 6 über den Anschluss 18 mit flüssigem Kulturmedium und phototrophen Organismen gefüllt, und zwar bis zu einem Füllstand dicht unterhalb der Stirnwand 5b. Bei gefülltem Reaktorraum 6 wird Gas, bevorzugt periodisch und stoßweise, in der Weise durch die Anschlüsse 13 in den Schlauch oder das Rohr 11 eingepumpt, dass es in Form großer, zusammenhängender Gasblasen aus den Schlitzen 15 austritt, die dann in jedem zweiten Kanal 9 in Richtung auf die Stirnwand 5b das Kulturmedium durchlaufen (siehe auch Figur 3) und senkrecht zur Erstreckungsrichtung der Zwischenwände 8 bevorzugt einen Durchmesser haben, der der Breite der Kanäle 9 entspricht oder nahezu entspricht. Gleichzeitig wird CO₂ durch den Anschluss 14 in den Schlauch oder das Rohr 11 eingepumpt und tritt in Form kleiner, feiner Gasbläschen in das Kulturmedium ein.

Durch die in jedem zweiten Kanal 9 aufsteigenden großen Gasblasen 20 wird innerhalb des Reaktorraums 6 ein spezielles Strömungsmuster erzeugt, das schematisch in Figur 3 dargestellt ist. Die aufsteigenden Gasblasen 20 erzeugen aufgrund des Airlift-Prinzips hydrostatisch eine Flüssigkeitsströmung 21 in den entsprechenden Kanälen 9 nach oben in Richtung auf die Stirnwand 5b und den Reaktorraumabschnitt 16. Aufgrund dieser Strömung 21 und der Luftblasen 20 entsteht in den übrigen Kanälen 9 eine Flüssigkeitsströmung 22 nach unten in Richtung auf die Stirnwand 5a. Die Flüssigkeitsströmung 21 reißt dabei nicht nur Flüssigkeit von den unteren Enden der Kanäle 9 mit (Pfeile 23 in Figur 3), sondern auch durch die Öffnungen 10 in den Zwischenwänden 8 aus den nicht begasten Kanälen 9 (Pfeile 24 in Figur 3). Der Reaktorraumabschnitt 7, der von der Stirnwand 5a bis zum dieser entgegengesetzten Ende der Zwischenwände 8 bzw. Kanäle 9 reicht, bildet demnach einen Strömungsleitabschnitt, in dem sich aufgrund der Anordnung der Zwischenwände 8 eine starke, durch die Kanäle 9 geführte Strömung ausbildet.

Durch die erreichbare hohe Strömungsgeschwindigkeit im Strömungsleitabschnitt 7, die über die Reaktorraumbreite abwechselnd auf- und absteigenden Flüssigkeitsströmungen und die weiter aufsteigenden Gasblasen 20 kann in dem gesamten Reaktorraumabschnitt 16 eine so starke dreidimensional turbulente Durchmischung stattfinden, dass die dort befindlichen Organismen in vorteilhafter Weise einen Hell-Dunkel-Zyklus mit einer Periodendauer von einigen Millisekunden durchlaufen.

## Patentansprüche

1. Bioreaktor zur Kultivierung phototropher Organismen in einem Reaktorraum mit:
einem lichtdurchlässigen quader- und plattenförmigen Gehäuse (2), in dessen Innerem der Reaktorraum (6) angeordnet ist, wobei das Gehäuse (2) zwei gegenüberliegende gröβere, die ausgedehnten Außenflächen des plattenförmigen Gehäuses (2) bildende Seitenwände (3), zwei gegenüberliegende kleinere Seitenwände (4) und zwei gegenüberliegende Stirnwände (5a, 5b) aufweist, die den Reaktorraum (6) begrenzen, und wobei der Abstand zwischen den kleineren Seitenwänden (4) die Breite des Reaktorraums (6) bestimmt, der Abstand zwischen den Stirnwänden (5a, 5b) die Länge des Reaktorraums (6) bestimmt und der Abstand zwischen den größeren Seitenwänden (3) die Dicke des Reaktorraums (6) bestimmt,
einer Vielzahl von Zwischenwänden (8), die parallel zu den kleineren Seitenwänden (4) und parallel zueinander in der Nähe einer ersten (5a) der beiden Stirnwände (5a, 5b) und beabstandet von der ersten Stirnwand (5a) angeordnet sind und sich über die gesamte Dicke des Reaktorraums (6) und über einen Teil der Länge des Reaktorraums (6) erstrecken, so dass ein Strömungsleitabschnitt (7) des Reaktorraums (6) mit einer Vielzahl von Strömungsleitkanälen (9) gebildet wird, von denen sich jeder zwischen zwei benachbarten Zwischenwänden (8) oder zwischen einer der kleineren Seitenwände (4) und einer benachbarten Zwischenwand (8) befindet, und zwischen den der ersten Stirnwand (5a) abgewandten Enden der Zwischenwände (8) und der zweiten (5b) der beiden Stirnwände (5a, 5b) ein freier Durchmischungsabschnitt (16) des Reaktorraums (6) vorgesehen ist, und
einer Gaszufuhreinrichtung zum Einbringen von Gas in jeden zweiten Strömungsleitkanal (9) in der Nähe der ersten Stirnwand (5a),
**dadurch gekennzeichnet, dass** sich der Durchmischungsabschnitt (16) über mindestens 50% der Länge des Reaktorraums (6) erstreckt und die Strömungsleitkanäle (9) eine Breite von mindestens 5 cm aufweisen.

2. Bioreaktor nach Anspruch 1, bei dem die Dicke des Reaktorraums (6) 1,5 bis 5 cm beträgt.

3. Bioreaktor nach Anspruch 2, bei dem die Dicke des Reaktorraums (6) mindestens 2,0 bis 3,0 cm beträgt.

4. Bioreaktor nach einem der vorhergehenden Ansprüche, bei dem die Breite der Strömungsleitkanäle (9) mindestens 15 cm beträgt.

5. Bioreaktor nach einem der vorhergehenden Ansprüche, bei dem entlang der Länge der Zwischenwände (8) in diesen eine Anzahl voneinander beabstandeter Öffnungen vorgesehen ist, durch die ein Flüssigkeitsaustausch zwischen benachbarten Strömungsleitkanälen (9) stattfinden kann.

6. Bioreaktor nach einem der vorhergehenden Ansprüche, bei dem die Gaszufuhreinrichtung ein oder mehrere Rohre (11) aufweist, die in dem Reaktorraum (6) zwischen der ersten Stirnwand (5a) und den dieser zugewandten Enden der Zwischenwände (8) verlaufen und Bohrungen oder Schlitze (10) aufweisen, durch die Gas in die jeweiligen Strömungsleitkanäle (9) abgegeben wird.

7. Bioreaktor nach Anspruch 6, bei dem die Gaszufuhreinrichtung angepasst ist, um bei mit flüssigem Kulturmedium und phototrophen Organismen befülltem Reaktorraum (6) Gas in Form einzelner Gasblasen (20) in die jeweiligen Strömungsleitkanäle (9) einzubringen, wobei die Gasblasen (20) einen großen Durchmesser von mindestens 3 cm aufweisen.

8. Bioreaktor nach Anspruch 6 oder Anspruch 7, bei dem die Gaszufuhreinrichtung angepasst ist, um das Gas in der Weise stoßweise in die entsprechenden Strömungsleitkanäle (9) einzubringen, dass erst dann eine neue Gasblase (20) erzeugt wird, wenn die vorhergehende Gasblase (20) das Kulturmedium in dem Reaktorraum (6) vollständig durchlaufen hat.

9. Bioreaktor nach einem der vorhergehenden Ansprüche, bei dem die Länge des Reaktorraums (6) größer als seine Breite ist.

10. Bioreaktor nach einem der vorhergehenden Ansprüche, bei dem im Durchmischungsabschnitt (16) eine Vielzahl voneinander beabstandeter Säulen (17) angeordnet ist, die jeweils die beiden großen Seitenwände (3) des Gehäuses (2) miteinander verbinden und jeweils so ausgebildet sind, dass sie sich in Richtung auf mindestens eines ihrer beiden Enden verjüngen.

11. Verfahren zur Kultivierung phototropher Organismen in einem Bioreaktor nach einem der vorhergehenden Ansprüche, das die folgenden Schritte aufweist:
Einbringen von flüssigem Kulturmedium in den Reaktorraum (6) eines Bioreaktors (1) nach einem der vorhergehenden Ansprüche,
Einbringen phototropher Organismen in den Reaktorraum (6), Bestrahlung einer großen Seitenwand (3) des Gehäuses (2) des Bioreaktors (1) mit Licht und
Einbringen von Gas in jeden zweiten Strömungsleitkanal (9) mit Hilfe der Gaszufuhreinrichtung, wobei das Gas in Form einzelner Blasen (20) eingebracht wird, deren großer Durchmesser mindestens 3 cm beträgt.

12. Verfahren nach Anspruch 11, bei dem das Gas mit Hilfe der Gaszufuhreinrichtung so eingebracht wird, dass die Geschwindigkeit der das Kulturmedium in den Strömungsleitkanälen (9) durchlaufenden Gasblasen (20) mindestens 0,5 m/sec beträgt.

13. Verfahren nach Anspruch 11 oder Anspruch 12, bei dem das Gas durch eine Stirnwand (5a) mit einem Verhältnis von Volumenstrom zu Fläche der Stirnwand, über die das Gas eingebracht wird, von mindestens 0,02 m/sec eingebracht wird.

14. Verfahren nach einem der Ansprüche 11 bis 13, bei dem das Gas in der Weise stoßweise in die entsprechenden Strömungsleitkanäle (9) eingebracht wird, dass erst dann eine neue Gasblase (20) erzeugt wird, wenn die vorhergehende Gasblase (20) das Kulturmedium in dem Reaktorraum (6) vollständig durchlaufen hat.

## Claims

1. A bioreactor for cultivating phototrophic organisms in a reactor chamber, comprising:
a translucent rectangular or plate-shaped housing (2) in the interior of which the reactor chamber (6) is disposed, wherein the housing (2) has two opposite major side walls (3) forming the extended outer surfaces of the plate-shaped housing (2), two opposite minor side walls (4), and two opposite end walls (5a, 5b) confining the reactor chamber (6), and wherein the distance between the minor side walls (4) determines the width of the reactor chamber (6), the distance between the end walls (5a, 5b) determines the length of the reactor chamber (6) and the distance between the major side walls (3) determines the thickness of the reactor chamber (6),
a plurality of intermediate walls (8) which are disposed parallel to the minor side walls (4) and parallel to one another in the vicinity of a first one (5a) of the two end walls (5a, 5b) and spaced from the first end wall (5a), and which extend over the entire thickness of the reactor chamber (6) and over a part of the length of the reactor chamber (6), so that a flow conducting section (7) of the reactor chamber (6) is formed having a plurality of flow conducting channels (9), each of which being located between two adjacent intermediate walls (8) or between one of the minor side walls (4) and an adjacent intermediate wall (8), and so that between the ends of the intermediate walls (8) facing away from the first end wall (5a) and the second (5b) of the two end walls (5a, 5b) a free mixing section (16) of the reactor chamber (6) is provided, and
a gas supply means for introducing gas into every other flow conducting channel (9) in the vicinity of the first end wall (5a),
**characterized in that** the mixing section (16) extends over at least 50 % of the length of the reactor chamber (6) and the flow conducting channels (9) have a width of at least 5 cm.

2. The bioreactor according to claim 1, wherein the thickness of the reactor chamber (6) is 1.5 to 5 cm.

3. The bioreactor according to claim 2, wherein the thickness of the reactor chamber (6) is at least 2.0 to 3.0 cm.

4. The bioreactor according to any of the preceding claims, wherein the width of the flow conducting channels (9) is at least 15 cm.

5. The bioreactor according to any of the preceding claims, wherein a number of spaced apertures are provided in the intermediate walls (8) along their length, through which apertures a liquid exchange can take place between adjacent flow conducting channels (9).

6. The bioreactor according to any of the preceding claims, wherein the gas supply means comprises one or more tubes (11) extending in the reactor chamber (6) between the first end wall (5a) and the ends of the intermediate walls (8) facing said first end wall (5a) and having bores or slits (10) through which gas is released into the respective flow conducting channels (9).

7. The bioreactor according to claim 6, wherein the gas supply means is adapted for introducing gas in the form of individual gas bubbles (20) into the respective flow conducting channels (9) when the reactor chamber (6) is filled with liquid culture medium and phototrophic organisms, wherein the gas bubbles (20) have a large diameter of at least 3 cm.

8. The bioreactor according to claim 6 or claim 7, wherein the gas supply means is adapted for intermittently introduce the gas into the corresponding flow conducting channels (9) in such a manner, that a new gas bubble (20) is generated only once the preceding gas bubble (20) has passed completely through the culture medium in the reactor chamber (6).

9. The bioreactor according to any of the preceding claims, wherein the length of the reactor chamber (6) is larger than its width.

10. The bioreactor according to any of the preceding claims, wherein a plurality of spaced posts (17) are disposed in the mixing section (16), each of which interconnecting the two major side walls (3) of the housing (2) and each of which being formed such that they taper towards at least one of their two ends.

11. A method for cultivating phototrophic organisms in a bioreactor according to any of the preceding claims, comprising the following steps:
introducing liquid culture medium into the reactor chamber (6) of a bioreactor (1) according to any of the preceding claims,
introducing phototrophic organisms into the reactor chamber (6),
illuminating one major side wall (3) of the housing (2) of the bioreactor (1) with light, and
introducing gas into every other flow conducting channel (9) by means of the gas supply means, wherein the gas is introduced in the form of individual bubbles (20), the large diameter of which being at least 3 cm.

12. The method according to claim 11, wherein the gas is introduced by means of the gas supply means, such that the velocity of the gas bubbles (20) passing through the culture medium in the flow conducting channels (9) is at least 0.5 m/sec.

13. The method according to claim 11 or claim 12, wherein the gas is introduced through an end wall (5a) with a ratio of volume flow to the area of the end wall via which the gas is introduced of at least 0.02 m/sec.

14. The method according to any of claims 11 to 13, wherein the gas is introduced into the respective flow conducting channels (9) intermittently in such a manner, that a new gas bubble (20) is generated only once the preceding gas bubble (20) has passed completely through the culture medium in the reactor chamber (6).

## Revendications

1. Bioréacteur pour la culture d'organismes phototrophiques dans un espace de réacteur avec :
un bâti (3) en forme de parallélépipède et de plaque, perméable à la lumière, dans l'intérieur duquel l'espace de réacteur (6) est placé, le bâti (2) présentant deux parois latérales opposées plus grandes (3) qui forment les surfaces extérieures étendues du bâti en forme de plaque (2), deux parois latérales opposées plus petites (4) et deux parois frontales opposées (5a, 5b) qui délimitent l'espace de réacteur (6) et la distance entre les parois latérales plus petites (4) définissant la largeur de l'espace du réacteur (6), la distance entre les parois frontales (5a, 5b) définissant la longueur de l'espace du réacteur (6) et la distance entre les parois latérales plus grandes (3) définissant l'épaisseur de l'espace du réacteur (6),
une multitude de parois intermédiaires (8) qui sont placées parallèlement aux parois latérales plus petites (4) et parallèlement les unes aux autres à proximité d'une première (5a) des deux parois frontales (5a, 5b) et en étant espacées de la première paroi frontale (5a) et en s'étendant sur toute l'épaisseur de l'espace de réacteur (6) et sur une partie de la longueur de l'espace de réacteur (6) si bien qu'une section conductrice de courant (7) de l'espace de réacteur (6) est formée avec une multitude de canaux conducteurs de courant (9), dont chacun se trouve entre deux parois intermédiaires voisines (8) ou entre l'une des parois latérales plus petites et une paroi intermédiaire voisine (8) et qu'une section de mélange libre (16) de l'espace de réacteur (6) est prévue entre les extrémités des parois intermédiaires qui sont détournées de la première paroi frontale (5a) et la seconde (5b) des deux parois frontales (5a, 5b)
et avec un dispositif d'alimentation en gaz pour introduire du gaz dans chaque deuxième canal conducteur de courant (9) à proximité de la première paroi frontale (5a)
**caractérisé en ce que** la section de mélange libre (16) s'étend sur au moins 50 % de la longueur de l'espace de réacteur (6) et que les canaux conducteurs de courant (9) présentent une largeur d'au moins 5 cm.

2. Bioréacteur selon la revendication 1 pour lequel l'épaisseur de l'espace de réacteur (6) est d'1,5 à 5 cm.

3. Bioréacteur selon la revendication 2 pour lequel l'épaisseur de l'espace de réacteur (6) est d'au moins 2,0 à 3 cm.

4. Bioréacteur selon l'une des revendications précédentes pour lequel la largeur des canaux conducteurs de courant (9) est d'au moins 15 cm.

5. Bioréacteur selon l'une des revendications précédentes pour lequel un certain nombre d'ouvertures espacées les unes des autres est prévu le long de la longueur des parois intermédiaires (8), ouvertures par lesquelles un échange de liquide peut avoir lieu entre des canaux conducteurs de courant (9) voisins.

6. Bioréacteur selon l'une des revendications précédentes pour lequel le dispositif d'alimentation en gaz présente un ou plusieurs tuyaux (11) qui se trouvent dans l'espace de réacteur (6) entre la première paroi frontale (5a) et les extrémités tournées vers celle-ci des parois intermédiaires (8) et qui présentent des forures ou des fentes (10) par lesquelles du gaz est délivré dans les canaux conducteurs de courant respectifs (9).

7. Bioréacteur selon la revendication 6 pour lequel le dispositif d'alimentation en gaz est adapté pour amener du gaz sous forme de bulles de gaz individuelles (20) dans lescanaux conducteurs de courant respectifs (9), lorsque l'espace de réacteur (6) est rempli d'un milieu de culture liquide et d'organismes phototrophiques, les bulles de gaz (20) présentant un grand diamètre d'au moins 3 cm.

8. Bioréacteur selon la revendication 6 ou 7 pour lequel le dispositif d'alimentation en gaz est adapté pour amener le gaz dans les canaux conducteurs de courant correspondants (9) de manière saccadée telle qu'une nouvelle bulle de gaz (20) n'est produite que lorsque la bulle de gaz précédente (20) a entièrement traversé le milieu de culture dans l'espace de réacteur (6).

9. Bioréacteur selon l'une des revendications précédentes pour lequel la longueur de l'espace de réacteur (6) est plus grande que sa largeur.

10. Bioréacteur selon l'une des revendications précédentes pour lequel une multitude de colonnes (17) espacées les unes des autres est placée dans la section de mélange (16), colonnes qui relient respectivement les deux grandes parois latérales (3) du bâti (2) l'une à l'autre et qui sont configurées chacune de telle manière qu'elles s'effilent en direction d'au moins l'une de leurs deux extrémités.

11. Procédé pour la culture d'organismes phototrophiques dans un bioréacteur selon l'une des revendications précédentes qui présente les étapes suivantes :
introduction d'un milieu de culture liquide dans l'espace de réacteur (6) d'un bioréacteur (1) selon l'une des revendications précédentes,
introduction d'organismes phototrophiques dans l'espace de réacteur (6),
irradiation d'une grande paroi latérale (3) du bâti (2) du bioréacteur (1) avec de la lumière et
introduction de gaz dans chaque deuxième canal conducteur de courant (9) à l'aide du dispositif d'alimentation en gaz, le gaz étant amené sous forme de bulles individuelles (20) dont le grand diamètre est d'au moins 3 cm.

12. Procédé selon la revendication 11 pour lequel le gaz est introduit à l'aide du dispositif d'alimentation en gaz de telle manière que la vitesse des bulles de gaz (20) qui traversent le milieu de culture dans les canaux conducteurs de courant (9) est d'au moins 0,5 m/s.

13. Procédé selon la revendication 11 ou 12 pour lequel le gaz est introduit à travers une paroi frontale (5a) avec un rapport de flux volumique à la surface de la paroi frontale, par laquelle le gaz est introduit, d'au moins 0,02 m/s.

14. Procédé selon l'une des revendications 11 à 13 pour lequel le gaz est introduit dans les canaux conducteurs de courant correspondants (9) de manière saccadée telle qu'une nouvelle bulle de gaz (20) n'est produite que lorsque la bulle de gaz précédente (20) a entièrement traversé le milieu de culture dans l'espace de réacteur (6).
